Europäisches Patentamt

(19) European Patent Office

Office européen des brevets

(11) Publication number: **0 439 263 A1**

# (12) EUROPEAN PATENT APPLICATION

(21) Application number: **91300218.4**

(22) Date of filing: **11.01.91**

(51) Int. Cl.⁵: **A61B 17/36, A61B 17/22**

(30) Priority: **12.01.90 US 463741**
**02.08.90 US 563594**

(43) Date of publication of application:
**31.07.91 Bulletin 91/31**

(84) Designated Contracting States:
**AT DE FR GB IT SE**

(71) Applicant: **LASERSCOPE**
**3052 Orchard Drive**
**San Jose California 95134 (US)**

(72) Inventor: **Grantz, Alan L.**
**2336 Silveria Court**
**Santa Clara, California 95054 (US)**
Inventor: **Gollnick, David A.**
**20884 Birch Street**
**Hayward, California 94541 (US)**

(74) Representative: **Jones, Ian et al**
**W.P. THOMSON & CO. High Holborn House**
**52-54 High Holborn**
**London WC1V 6RY (GB)**

(54) Instrumentation clamp.

(57) A clamp for clamping instrumentation uses a resilient tube (507) and a compression housing (600) operatively associated with the tube for providing a compressive force on the tube. The compressive force is distributed generally equally over the length of the tube so that instrumentation disposed through the tube is substantially secured within the housing with minimal deformation. The clamp is especially adaptable to fragile instrumentation, such as optical fibers, which typically are clamped for manipulation. In one embodiment, the clamp is permanently attached to one instrument, while clamping another instrument for simultaneous manipulation. In another embodiment, the instrumentation clamp can couple to one instrument temporarily, while clamping another instrument. This embodiment is designed to be self contained and readily adaptable for multiple uses.

FIGURE 23A

EP 0 439 263 A1

# INSTRUMENTATION CLAMP

This invention relates to a clamping device for clamping an instrument, or an instrumentation clamp.

The invention is applicable especially to clamping instruments which are cylindrical in shape for example optical fibers for manipulation when using a laser. One particular application of an instrumentation clamp of the invention is in laser surgery, such as percutaneous diskectomy.

Reference is now made to Figures 1-4 of the accompanying drawings, in which :

Figure 1 is a posterior view of the lumbar vertebral column.

Figure 2 is an oblique view of a lumbar disc and inferior vertebrae.

Figures 3A is a sectional view of a herniated lumbar vertebrae and an associated nerve root.

Figure 3B is a sectional view of the vertebrae in Figure 3A after nucleus pulposus is removed.

Figure 4 is an oblique view illustrating the Nucleomtome Probe of the prior art.

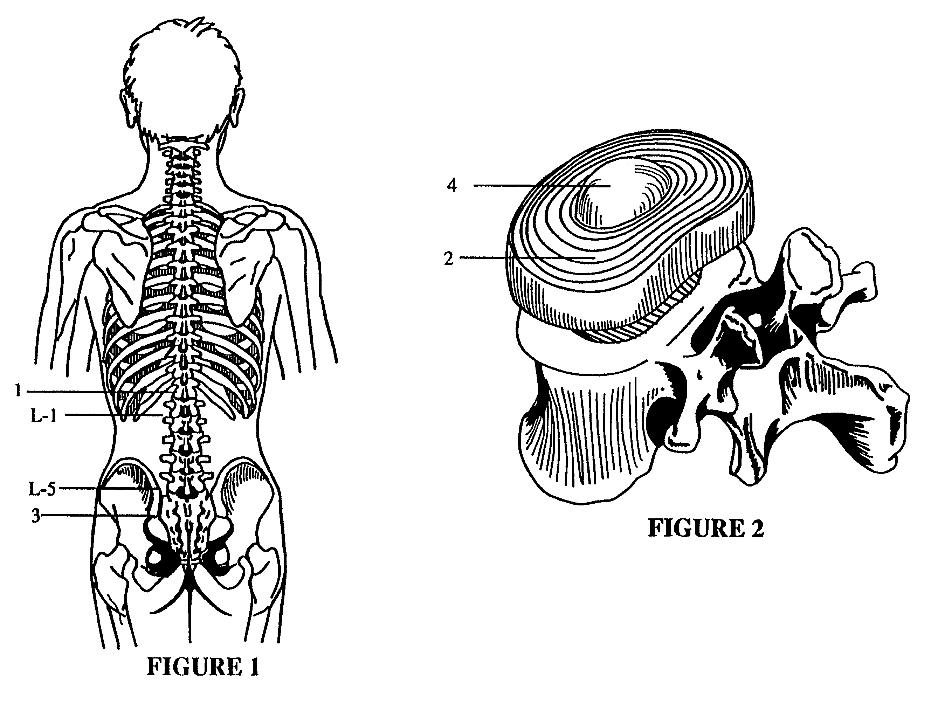

Mechanically assisted percutaneous lumbar diskectomy of the prior art is used as a treatment of leg pain (sciatica) resulting from herniated discs of the lumbar vertebral column. The lumbar vertebral column consists of five vertebrae extending superiorly to the transitional thoracic vertebrae (1) at a first end and extending inferiorally to the sacrum (3) at a second end, as illustrated in Figure 1. Between each lumbar vertebrae and between the lumbar and sacrum are cartilaginous discs. Each disc comprises an outer circular structure (annulus fibrosus) 2 which surrounds and tightly binds an inner gelatinous material (nucleus pulposus) 4 in the center, as illustrated in Figure 2. The annulus fibrosus 2 is made up of concentric fibers which appear to cross each other obliquely. No blood vessels or nerves penetrate the nucleus.

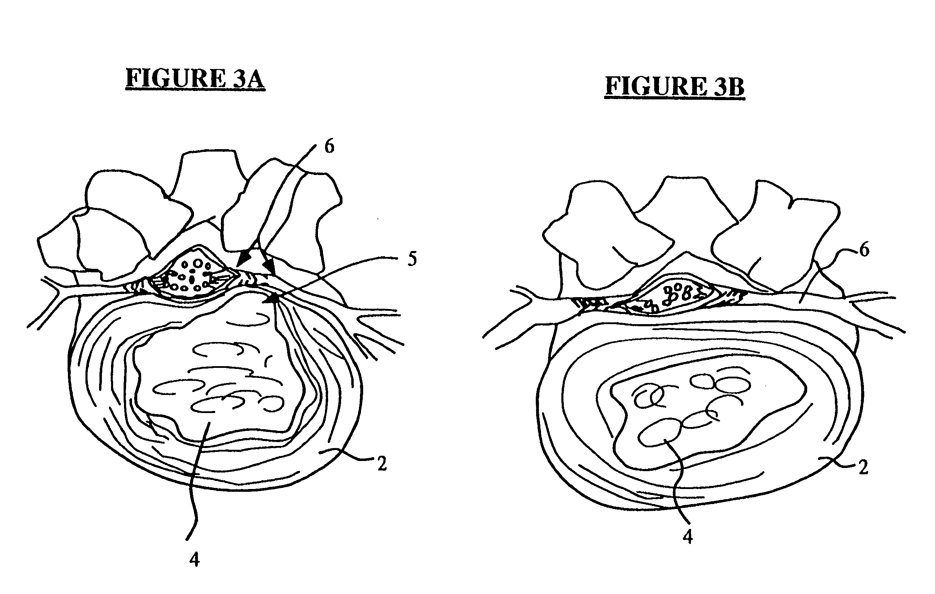

Usually with age, the fibers of the annulus begin to degenerate. The degeneration results in the tearing of individual fibers when the vertebral column is stressed. Torn fibers can form fenestrations which allow the nucleus pulposus to move through the fibers of the annulus and bulge 5 outward away from the nucleus. If the bulged disc presses upon an adjacent nerve root 6, sciatica may develop, as illustrated in Figure 3A.

It has been demonstrated that removing a portion of the nucleus with grasping forceps through a small cannula will produce good to excellent relief of pain in a majority of patients having symptoms indicative of sciatica. Once a portion of the nucleus is removed, the pressure against the nerve root causing the pain is relieved as the remaining nucleus contracts away from the pressure point, as illustrated in Figure 3B. Hijikata S., Yamagishi M., Nakayama T., Oomori K., "Percutaneous Diskectomy : A New Treatment for Lumbar Disc Herniation", J. Toden Hospital 1975 ; 5 : 5-13. Since the Hijikata et al. article, mechanical forceps for microlumbar and percutaneous lumbar diskectomy procedures have been developed related to relieving sciatica pain.

U.S. Patent No. 4,369,788, which issued in January 25, 1983 to Goald discloses a forceps device having an alligator jaw for microlumbar disc surgery. For microlumbar disc surgery, a one-inch incision is made in the patient into which the forceps are inserted and the surgery is performed.

U.S. Patent No. 4,545,374, which issued on October 8, 1985 to Jacobson discloses a method and instruments for performing percutaneous diskectomy using a knife to severe the disc nucleus and Rongeur forceps to remove the severed fragments of disc nucleus. The diskectomy tools are inserted through a cannula to the injured disc area.

The instrumentation and procedure taught by Jacobson require extensive manipulation of tools by the surgeon, that a more streamlined procedure using fewer tools would be desirable.

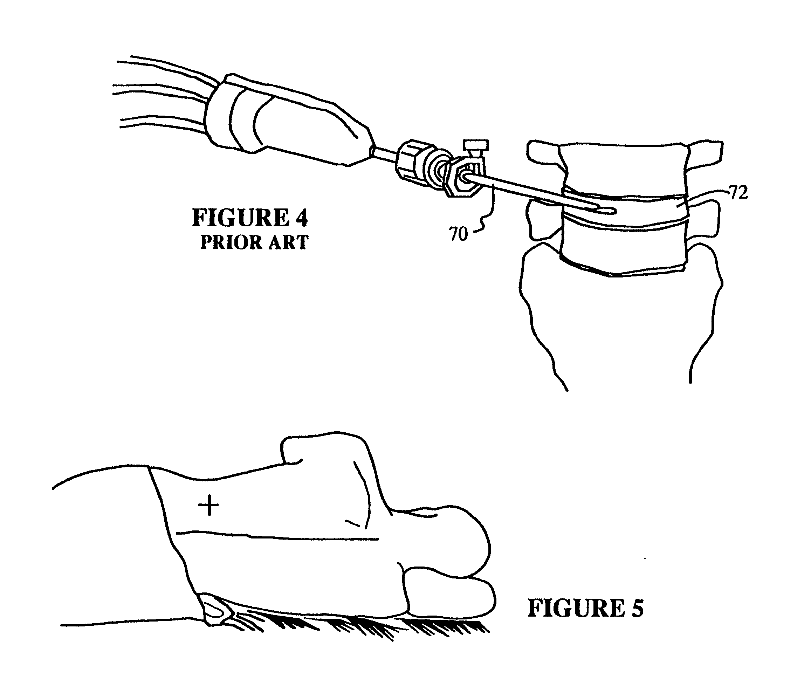

U.S. Patent No. 4,678,459 issued to Onik et al. on July 7, 1987 discloses using an irrigating, cutting and aspirating system for percutaneous surgery. Onik et al. disclose using a system for removing nucleus pulposus tissue which includes a probe and a guillotine type of cutting means for cutting the nucleus pulposus. The severed or cut fragments of nucleus pulposus are removed from the cutting means using an internal fluid irrigation system and a vacuum to aspirate the severed fragments out of the disc area, through the system, and out of the patient. This system provides for a relatively fast diskectomy procedure compared to the other prior art because nucleus pulposus can be fragmented and removed without the need to manipulate many small blades, knives and forceps, as described for the Jacobson patent 4,545,174. The probe and guillotine-type cutting means disclosed by Onik et al. is sold on the market as a Nucleotome Probe 70, as illustrated in Figure 4. This instrument is the most widely used instrument for percutaneous diskectomy. The Nucleotome Probe 70 is inserted into a cannula 73 and is locked into place on the cannula 73, as illustrated in Figure 4. When the Nucleotome Probe 70 is activated, the nucleus pulposus is cut into fragments which are removed with irrigation fluids and suction, all within the Nucleotome Probe 70. The Nucleotome Probe 70 is activated until no further material can be extracted. Once complete, the Nucleotome Probe 70 and cannula 73 are removed and the entry point is covered with a sterile bandage. The cutting and extracting process alone using the Nucleotome Probe 70 normally takes between 20 to 30 minutes.

It would be desirable if a laser technique and laser instrumentation were available to perform percutaneous

EP 0 439 263 A1

diskectomies so that nucleus pulposus from herniated discs could be vaporized using a laser in a safe and effective way which is faster than cutting and irrigating using the Nucleotome Probe and which would eliminate the need to cut and remove fragmented debris from the patient.

A means for clamping is provided which clamps and grips fragile instrumentation with minimal slippage and deformation. Each means for clamping can couple the clamped instrument to a second instrument for manipulation of both instruments simultaneously. In a first embodiment, the clamping means is permanently attached to a second instrument and in particular, the clamping means attaches instrumentation to an introducer tube. In a second embodiment, the clamping means can be temporarily coupled to the second instrument. The instrumentation clamp is self-contained and reusable. The means for coupling of the second embodiment incorporates standard temporary coupling means which makes the instrumentation clamp readily adaptable for multiple uses. The means for clamping works particularly well with the instrumentation described herein for percutaneous diskectomy using a laser.

According to the present invention, the means for clamping comprises a resilient tube and means operatively associated with the resilient tube for providing a compressive force. The means for providing a compressive force comprises a clamping portion having means for holding said resilient tube and having means for coupling to a second instrument ; and a clamp housing operatively secured to said means for holding said resilient tube, said clamp housing selectively compressing said means for holding against said resilient tube. The compressive force is distributed generally equally over the entire length of the tube so that instrumentation disposed through the tube is substantially secured with minimal deformation.

The means for clamping is especially adaptable to clamping optical wave guides such as optical fibers, which are typically clamped for manipulation. For example, optical fibers are clamped and secured to instrumentation which introduces the optical fiber into a patient for surgical procedures using a laser. The means for clamping according to the present invention can securely couple two instruments together for applications such as percutaneous diskectomy using a laser.

The invention is further described below, by way of example, with reference to the remaining Figures of the accompanying drawings, in which :

Figure 5 is a posterior view of a patient in a lateral decubitus position.

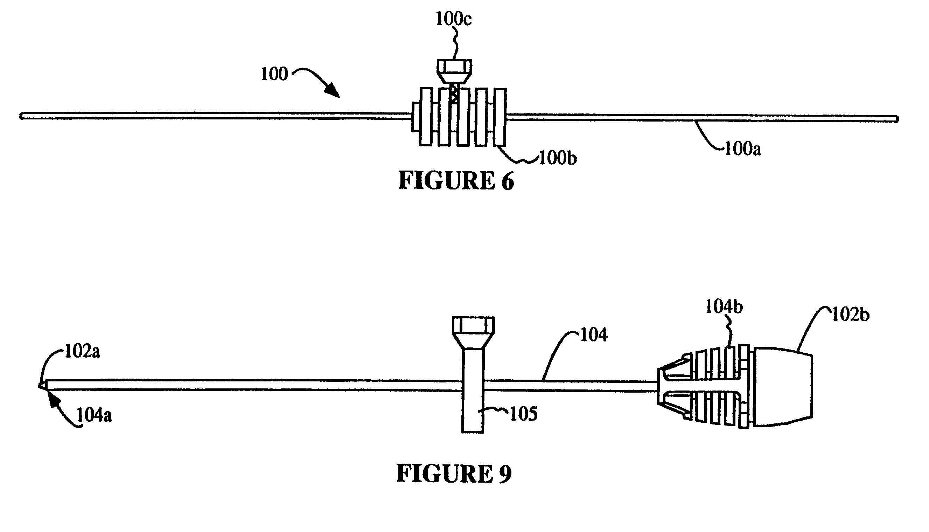

Figure 6 is a side view illustrating a probe used with the present invention.

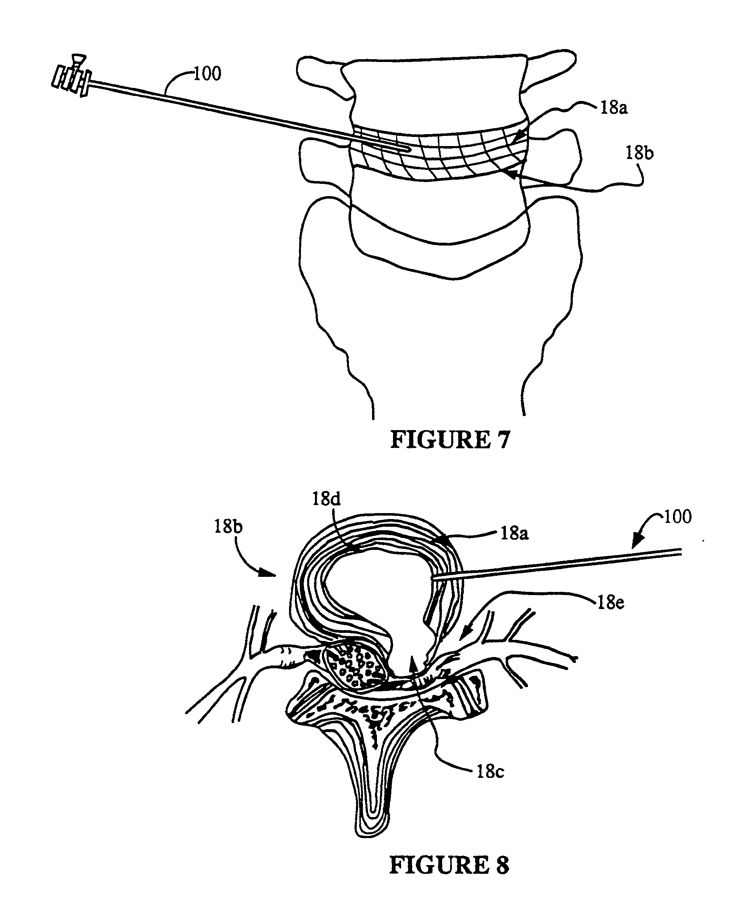

Figure 7 is an oblique view illustrating the probe inserted into a disc according to the present invention.

Figure 8 is a sectional view of a herniated disc and associated nerve root having the probe inserted thereinto according to the present invention.

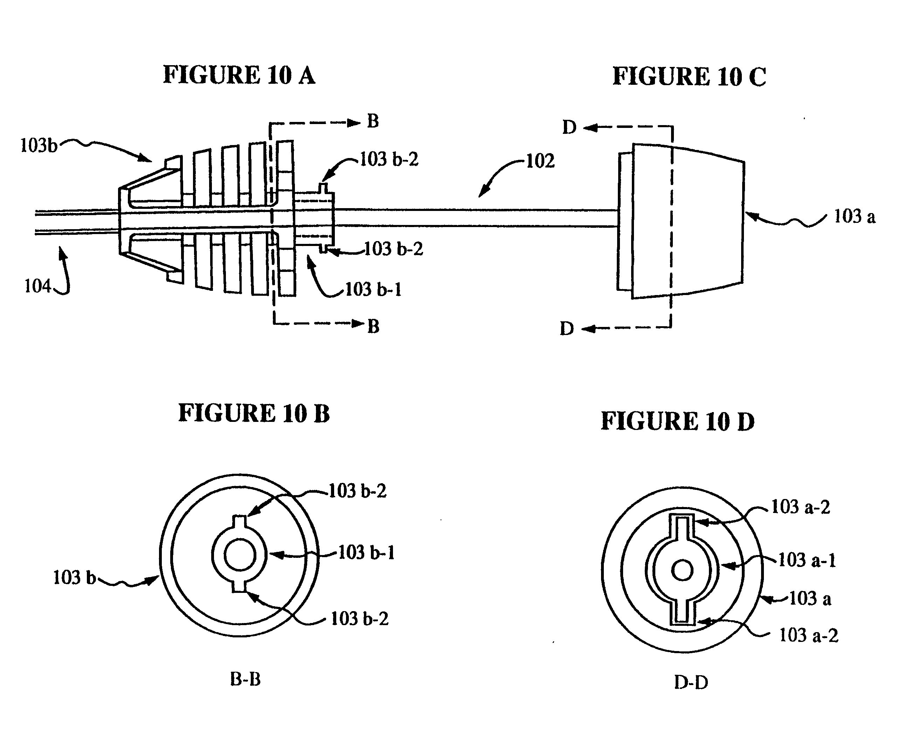

Figure 9 is a side view of a cannula having a dilator inserted thereinto used with the present invention.

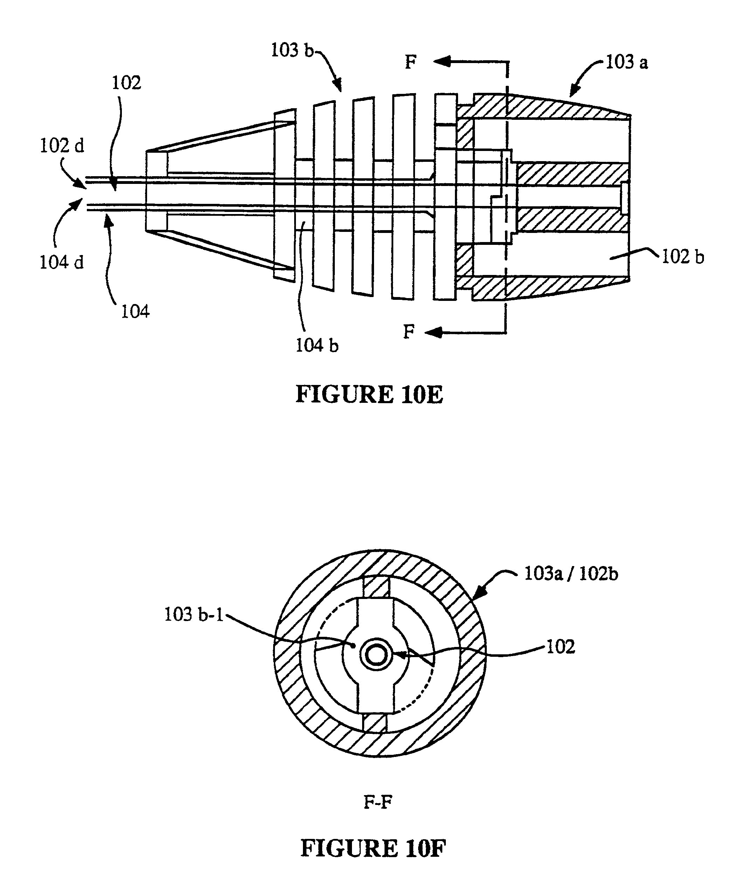

Figures 10A-10E are sectional and plan views of a bayonet type lock fitting used with the present invention.

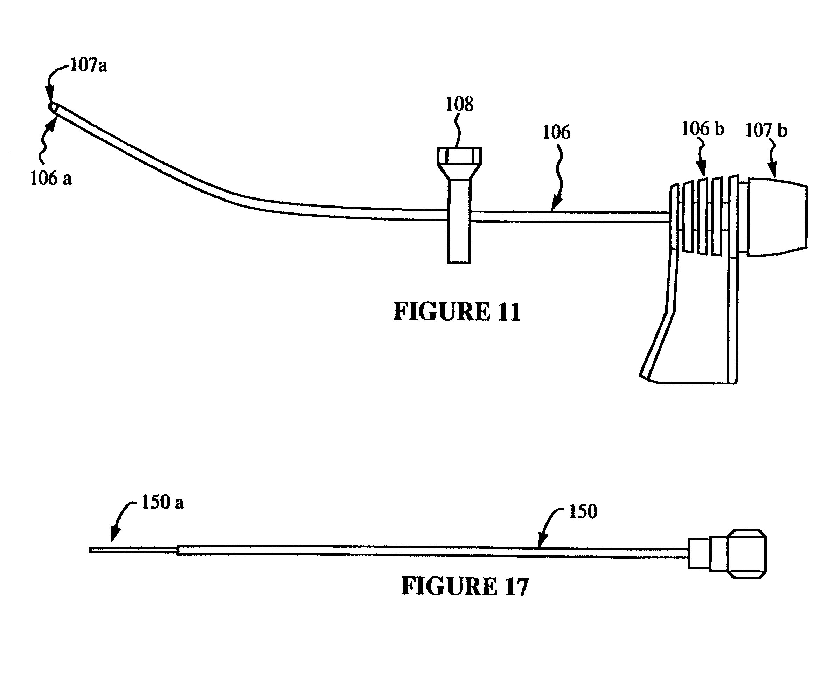

Figure 11 is a side view illustrating a curved cannula used with the present invention.

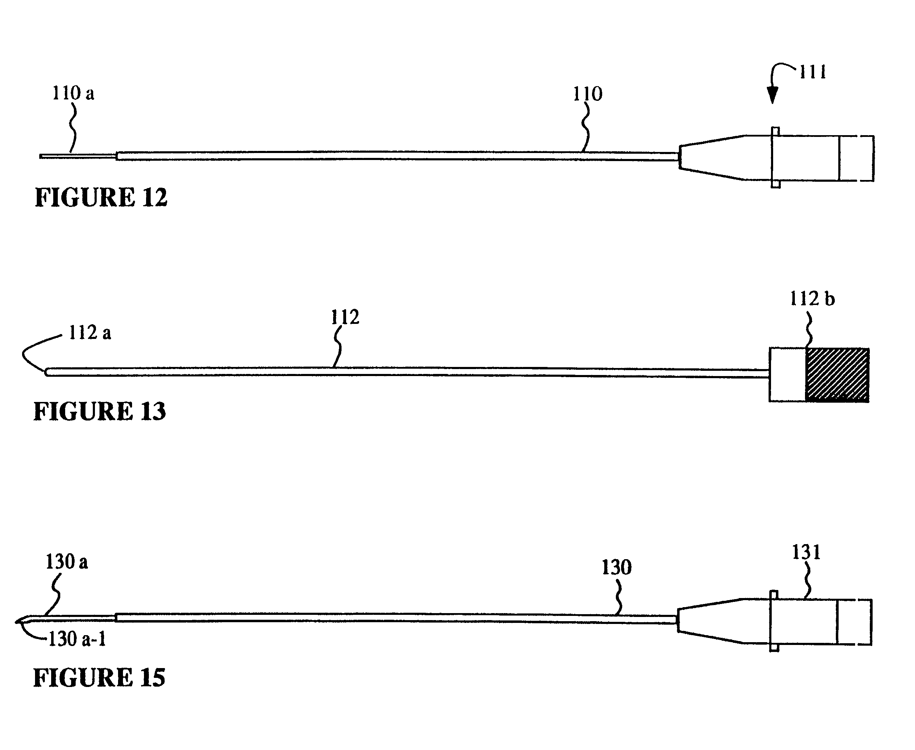

Figure 12 is a side view illustrating an introducer means according to the present invention.

Figure 13 is a side view illustrating a stylet used with the present invention.

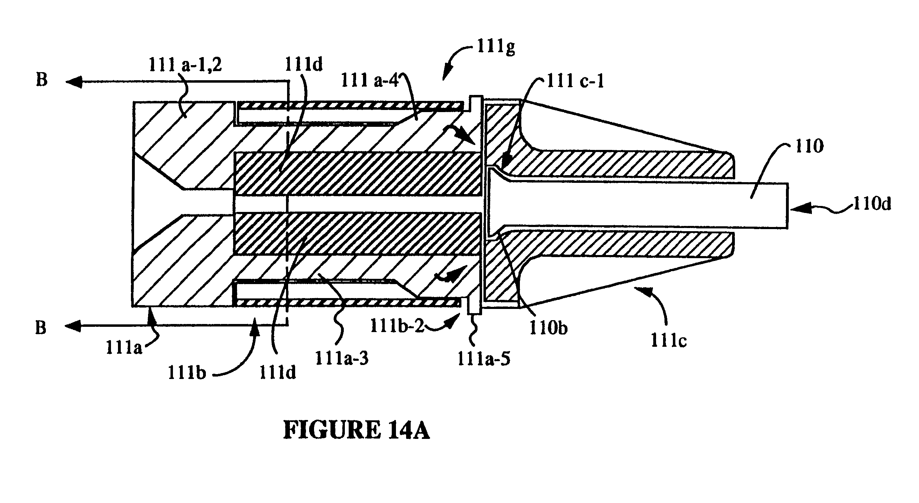

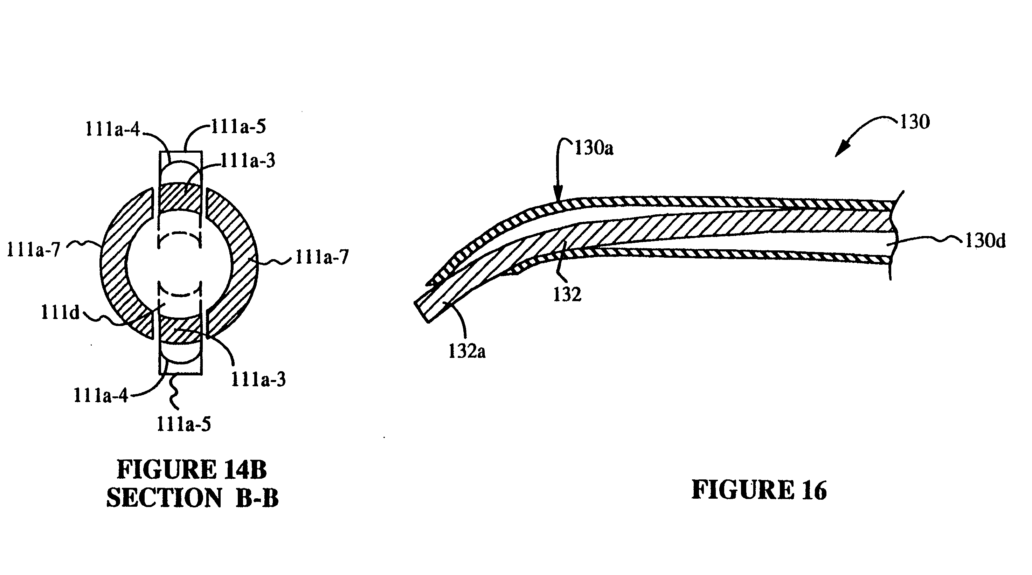

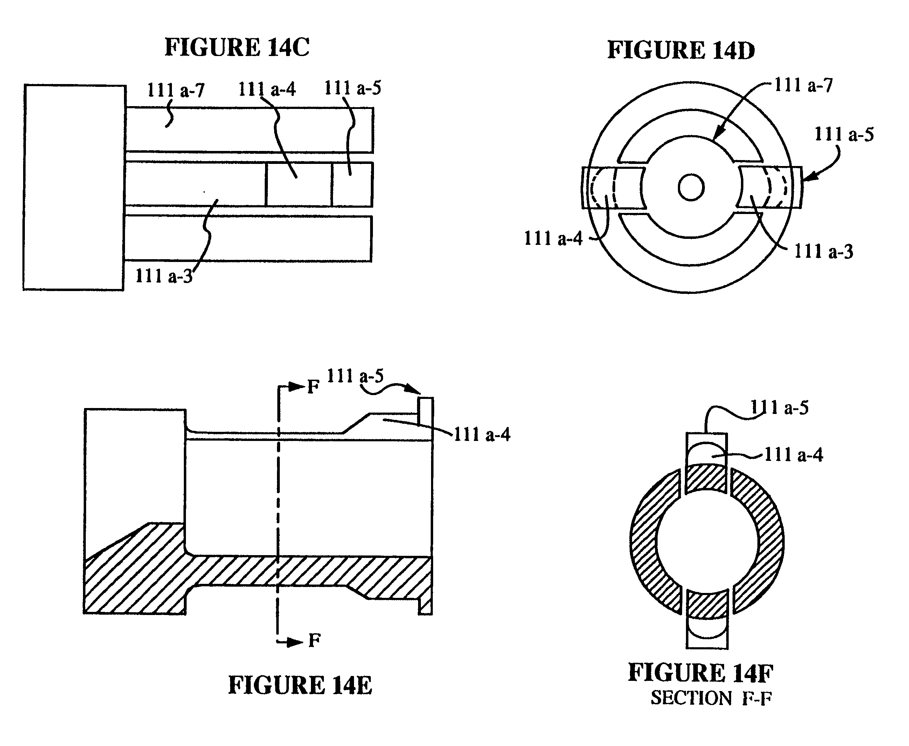

Figure 14A-H are sectional views illustrating a clamping means according to the present invention.

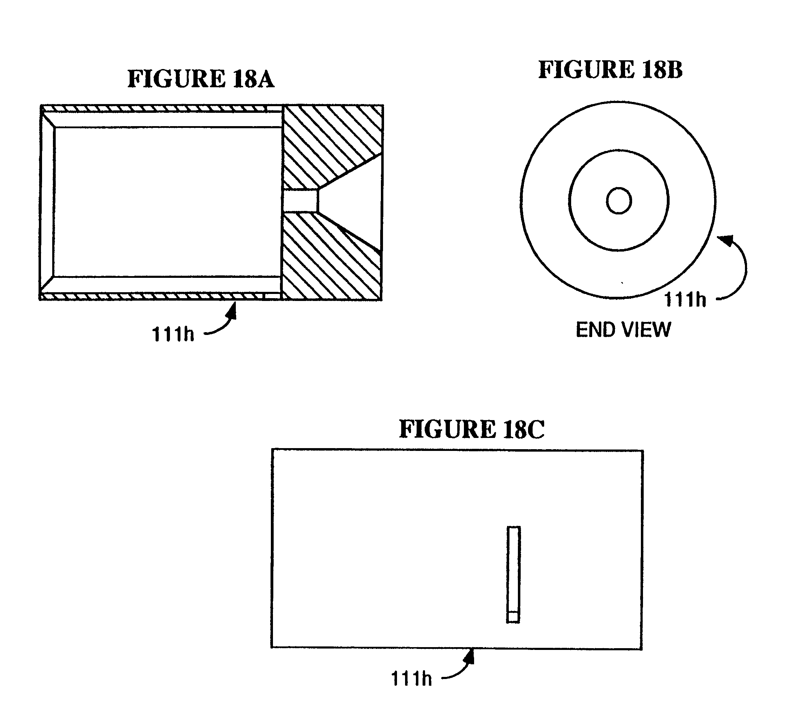

Figure 15 is a side view illustrating an introducer means having a formed end according to the present invention.

Figure 16 is an enlarged sectional view illustrating an optical guiding means emanating from the formed end of introducer means according to the present invention.

Figure 17 is a side view of an irrigation/aspiration cannula used with the present invention.

Figures 18A-C are sectional views illustrating a position indicator means according to the present invention.

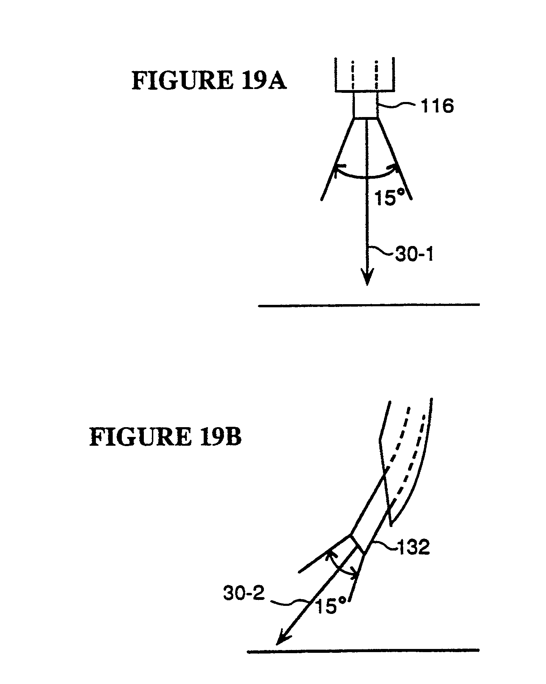

Figures 19A-B are plan views illustrating the first line of a laser beam.

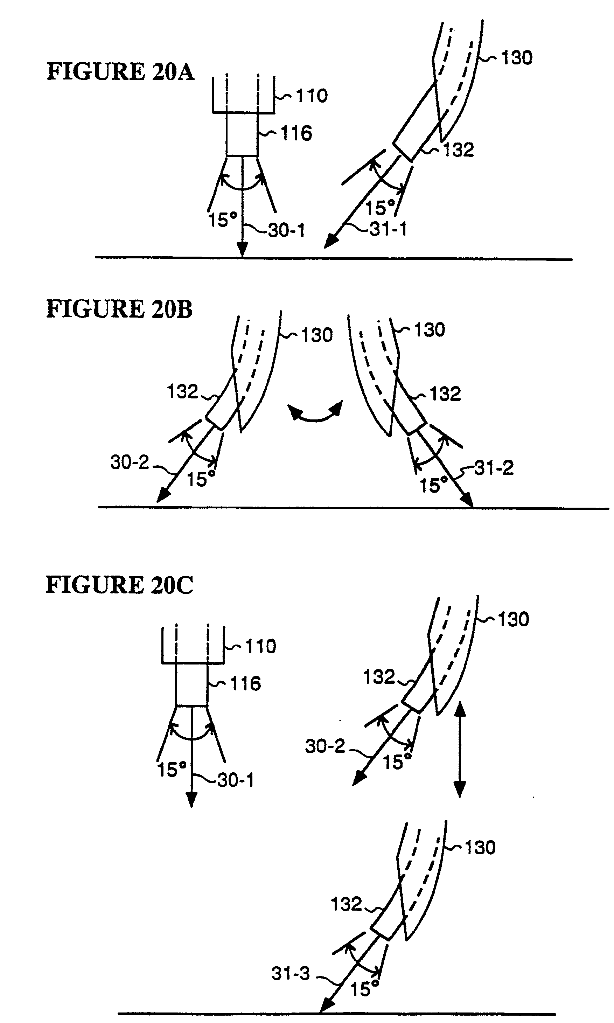

Figures 20A-C are plan views illustrating the second line of a laser beam.

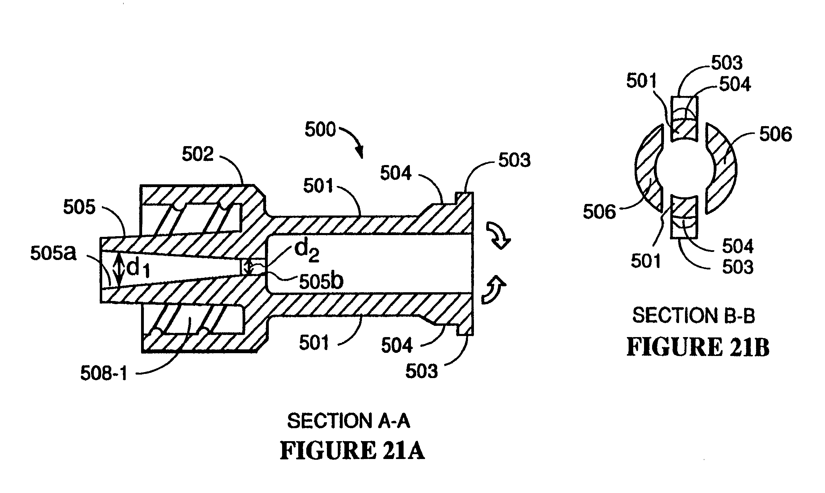

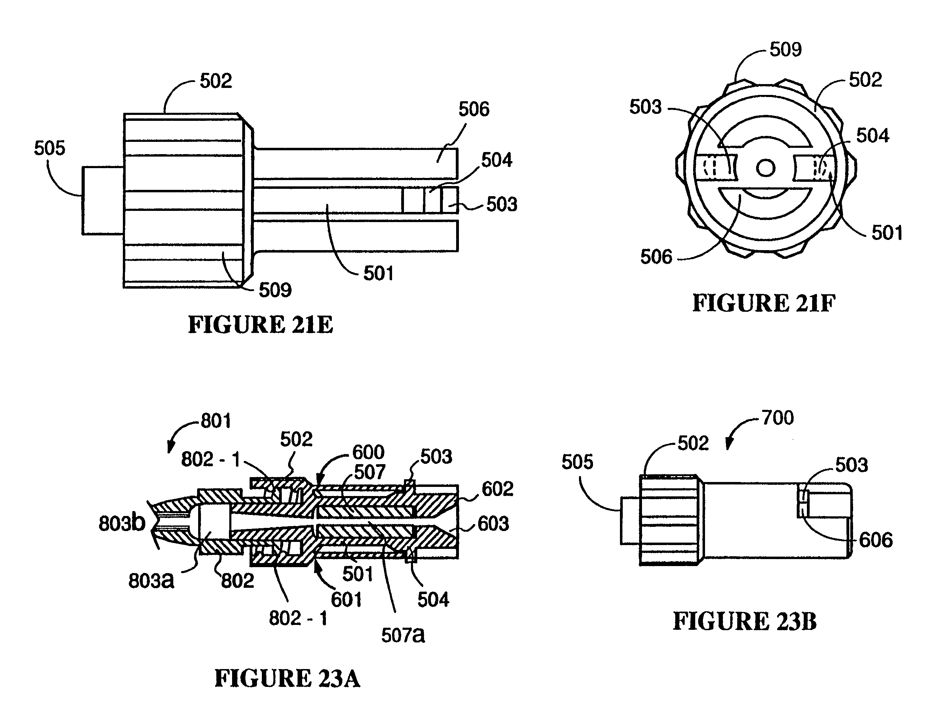

Figures 21A-21F illustrate sectional and plan views of the clamping portion to the second embodiment of the clamping means according to the present invention.

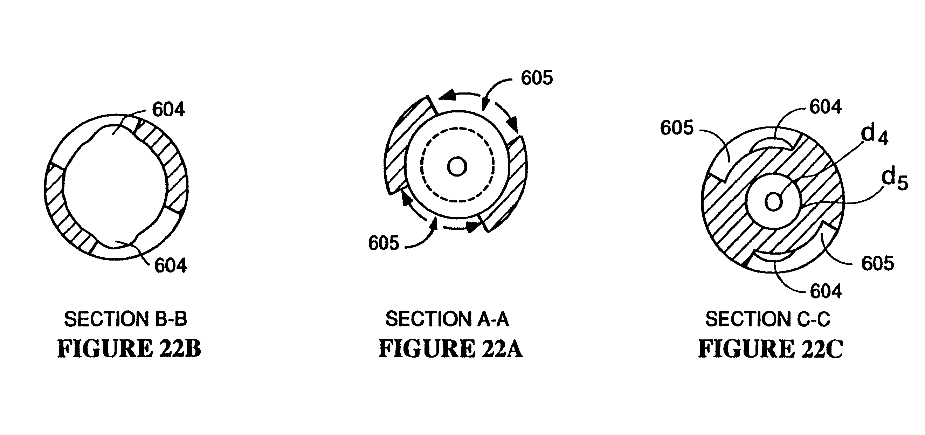

Figures 22A-22G illustrate sectional and plan views of the clamp housing to the second embodiment of the clamping means according to the present invention.

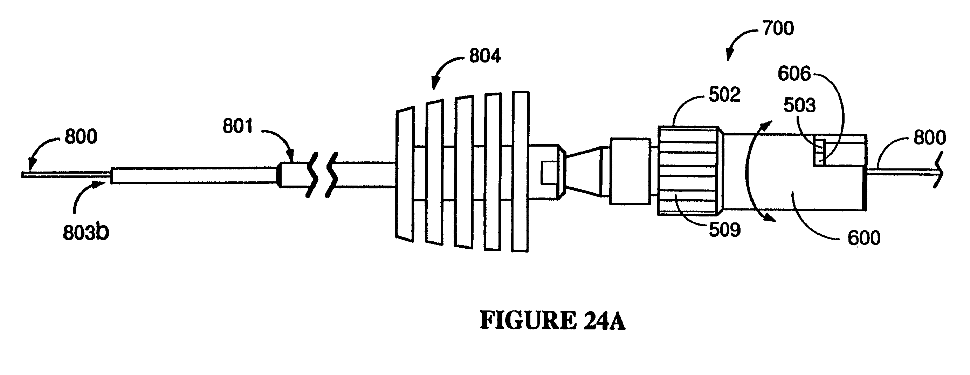

Figure 23A is a sectional view and Figure 23B is a plan view illustrating the assembled instrumentation clamp of the second embodiment of the clamping means according to the present invention.

Figure 24A illustrates the instrumentation clamp of Figure 23B associated with both a first instrument and a second instrument, according to the preferred embodiment of the clamping means.

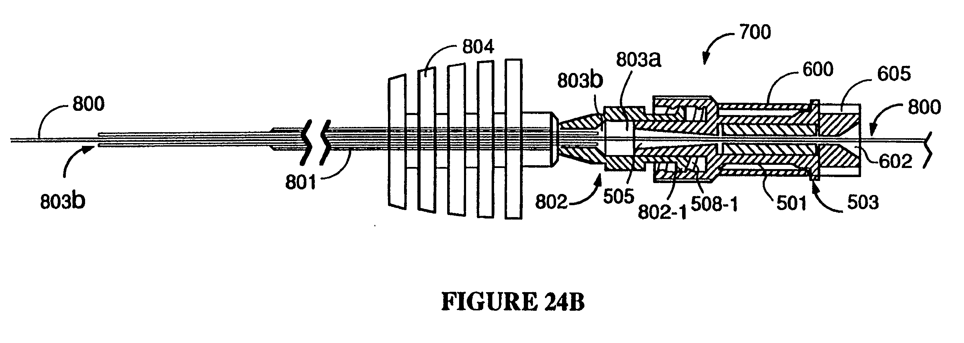

Figure 24B illustrates a cross-sectional view of Figure 24A.

A percutaneous diskectomy procedure using a laser is designed for patients commonly showing evidence clinically and radiologically of nerve root impingement. Conventionally, physical examination of the patient should reveal leg pain greater than back pain and signs of nerve root irritation consistent with a herniated disc. Radiographically, the patient should exhibit a focal herniation or bulge that shows an impression on the thecal

3

sac which does not occupy more than fifty percent of the thecal sac. Also, the radiographic results should correlate with the patient's symptomatology.

Vaporization of nucleus pulposus material according to the present invention suggests that the operative tools be inserted at an entry site on the same side of the patient's body that the herniation or other affliction is evident. The path of entry to the afflicted disc should avoid going through the psoas muscle since the lumbar plexus has numerous fibers which traverse the muscle. Conventionally, a computed tomograph (CT) scan slice of the whole abdomen through the involved disc is quite helpful for determining the entry path.

The safety of the procedure relies on radiologic localization and guidance of the instruments into the disc and a C-arm fluoroscope with image intensification, known in the art, can provide clear and sharp images in anteroposterior, lateral and oblique views.

Typically, the patient undergoing a percutaneous diskectomy procedure is positioned on a fluoroscopic table, which is known in the art, in a lateral decubitus position, as illustrated in Figure 5. The patient must be stabilized to prevent rotation of the patient's shoulders and hips during the procedure. Using a fluoroscope, the sacrum is identified and located, the afflicted disc is located, and as illustrated in Figure 5, a posterolateral entry point is selected. The entry point typically is 8-12 centimeters from the midline and both parallel and midway between the end plates of the afflicted disc, as determined using a measuring scale. Local anesthetic is used to anesthetize the area to be operated on which is administered typically with a long spinal needle.

At this point in the patient preparation procedure, the percutaneous diskectomy procedure using a laser and means for inserting instrumentation according to the present invention is described.

First, one end of a semi-rigid trocar or probe 100, which is preferably 18 gauge Birmingham Wire Gauge (BWG), is inserted at the entry point once the anesthetic has taken effect. Probe 100 has an elongated body 100a and has a standard tube clamp 100b with a threaded lock 100c connected to probe 100, as illustrated in Figure 6.

Clamp 100b is removable from body 100a by loosening lock 100c and sliding clamp 100b in either direction beyond the end of probe 100. Clamp 100b serves as a handle to hold probe 100 while it is inserted into the patient. Clamp 100b is removed for subsequent steps described below. Clamp 100b may be made of plastic, for example acrylonitrile-butadiene-styrene (ABS) plastic or preferably polycarbonate plastic, or metal, preferably stainless steel.

Probe 100 is preferably made of stainless steel, for example type 304 or an equivalent, No. 3 temper. The inserted end of probe 100 has a sharp tip and is guided into the damaged or herniated disc area 18c with radiologic localization and guidance, preferably using the C-arm fluoroscope with image intensification, as described above. Probe 100 is inserted until the inserted end punctures through the annulus fibrosus 18a of the disc 18b, as illustrated in Figures 7 and 8. While probe 100 is in place, extending from the disc area to outside the patient's body, a cannula 104 having a dilator 102 inserted thereinto is inserted over probe 100 at the exterior end and into the probed disc area 18c. One end of dilator 102 (102b) and cannula 104 (104b) remain on the exterior of the patient.

Cannula 104 and dilator 102 are preferably 12 gauge stainless steel tubing, for example type 304, No. 3 temper (full hard). Stainless steel tubing may be purchased at any stainless steel tubing supplier, for example Poper and Sons, N.Y. Dilator 102 preferably is longer than cannula 104 and has a tapered end 102a which extends beyond the end 104a of cannula 104, as illustrated in Figure 9, for ease of insertion over probe 100 through the patient's skin. Dilator 102 has bore 102d which extends through the center of dilator 102 along its length. Probe 100 fits within bore 102d of dilator 102. Cannula 104 is preferably a straight tubular member having a central bore 104d, which extends along its length. Dilator 102 and probe 100 fit within bore 104d of cannula 104.

Cannula 104 and dilator 102 have locking means 103 (103 mechanism not shown in Figure 9) for locking dilator 102 to cannula 104 at end 104b and 102b, respectively, and a locking stabilizer 105, as illustrated in Figure 9. Locking means 103 is preferably a bayonet-fitting locking mechanism, as illustrated in Figures 10A-10E as portions 103a and 103b. Dilator 102 has portion 103a and cannula 104 has portion 103b of bayonet-fitting locking mechanism 103. Portion 103b on cannula 104 has a segmented body and flared legs for gripping and preferably projection 103b-1 having two laterally extending flanges 103b-2 which oppose one another. Portion 103a on dilator 102 preferably has aperture 103a-1 and sockets 103a-2. Aperture 103a-1 is at least as deep as the distance projection 103b-1 projects. Sockets 103a-2 oppositely extend off aperture 103a-1 and are at least as deep as flanges 103b-2 are thick. Projection 103b-1 and flanges 103b-2 fit within aperture 103a-1 and sockets 103a-2, respectively. Once fitted together, end 102b of dilator 102 is turned clockwise thereby rotating dilator 102 within cannula 104 to lock locking portion 103a to locking portion 103b and thereby lock dilator 102 to cannula 104 using locking means 103. Locking means 103 can also be a luer lock, threaded screw lock, snap lock or a friction fitted lock, which are known in the art. Locking means 103 and stabilizer 105 can be made of plastic, ABS or preferably polycarbonate plastic, or metal, preferably stainless steel. In the preferred embodiment, means 103 and stabilizer 105 are made of a plastic which can withstand at least the stresses associated with gamma sterilization techniques without distortion. The plastic may also withstand the stresses associated with autoclaving and usage of ethylene oxide gas sterilization methods. Locking stabilizer 105 is adjustably located along the length of cannula 104 and serves to rest against the patient's skin when the cannula is properly placed.

In another embodiment, curved cannula 106 may be inserted into the patient instead of straight cannula 104. Cannula 106 is a curved tubular member having a locking dilator 107 and locking stabilizer 108, as illustrated in Figure 11. Curved cannula 106 is used in situations where the patient's afflicted area is within the lumbar 5-sacrum 1 region of the vertebral column, as shown in Figure 1.

Once dilator 102 and cannula 104 are confirmed, preferably fluoroscopically, to be embedded in the annulus fibrosus, dilator 102 is unlocked from locking mechanism 103 and removed. Dilator 102 is unlocked by turning portion 103a counterclockwise while holding portion 103b on cannula 104 stationary. As dilator 102 is withdrawn, cannula 104 is advanced forward to embed in the wall of the annulus approximately the distance equal to the difference in length of dilator 102 and cannula 104. Cannula 104 is secured by stabilizer 105 by unlocking the screw mechanism, sliding stabilizer 105 up against the patient's skin, and locking the screw. Probe 100 is removed once cannula 104 is secured.

Second, one end of a first introducer means or tube 110 for inserting instrumentation according to this invention is inserted into central bore 104d at the exterior end 104b of cannula 104. First introducer means 110 is a substantially straight elongated member preferably 14 gauge along most of its length and having a 17 gauge tip 110a at one end, as illustrated in Figure 12, and having a clamping means 111 at an opposite end for clamping to an optical guiding means, as is described below. In another embodiment, the first introducer means and means for clamping are separate devices. First introducer means 110 is metal, preferably type 304 stainless steel, No. 3 temper (full hard). Clamping means 111 can be plastic, preferably polycarbonate plastic or metal, preferably stainless steel. First introducer means 110 has a bore 110d which extends through the center of first introducer means 110 along its length.

When the one end 110a of first introducer means 110 is inserted into bore 104d of cannula 104, first introducer means 110 preferably has a stylet 112 extending therethrough. Stylet 112 is a long straight member, preferably 18 gauge stainless steel, having a sharp tip 112a at one end and a handle means 112b for handling stylet 112 at the opposite end, as illustrated in Figure 13. The sharp end 112a can be a conical-shaped tip, diamond shaped tip or beveled, for example. Sharp end 112a extends out of the inserted end 110a of the first introducer means and stylet 112 is clamped by clamping means 111 on first introducer means 110 at handle means end 112b. The clamping mechanism for clamping means 111 will be described below. Stylet 112 is longer than and narrower in diameter than first introducer means 110 and fits within bore 110d of first introducer means 110. Stylet 112 can be adapted to also lock with the means for clamping at handle means 112b either with a luer lock, threaded screw lock, snap lock, or friction fit lock, depending on the embodiment of the means for clamping.

Because the sharp tip extends out of the inserted end 110a of first introducer means 110, stylet 112 contacts the outer wall of the nucleus 18d and enters into the nucleus with its sharp tip 112a, leaving a small opening. Since the nucleus is a soft gelatinous material, stylet 112 enters the nucleus with minimal resistance and the inserted end 110a of first introducer means 110 is placed in the nucleus. Stylet 112 is removed from the nucleus through the first introducer means 110, and the first introducer means or tube 110 is left in place for introducing instrumentation into the nucleus.

Third, one end of a first optical guiding means 116 for guiding laser light is inserted through bore 110d of first introducer means 110 after stylet 112 is removed. First optical guiding means 116 is inserted until it emanates from end 110a of first introducer means into the small opening made by stylet 112.

First optical guiding means 116 is preferably an optical fiber or a hollow optical wave guide (both not shown), depending on the embodiment. In one embodiment, an optical fiber can be used which is preferably 400 micrometers in inner diameter and 600 micrometers in outer diameter and is made of quartz. In another embodiment, a hollow optical wave guide can be used which can be made from metal or preferably ceramic. The hollow waveguide is rigid compared to the optical fiber.

First optical guiding means 116 passes through first introducer means 110 and into the nucleus 18d at a first end and is connected to a first laser means for producing laser light at a second end outside of the patient. First optical guiding means 116 can have a position indicator means 111h for indicating a preset distance optical guiding means 116 must extend out of first introducer means 110 at end 110a, depending on the embodiment. Positioning indicator means 111h, which is described below, is illustrated in Figures 18A-18C and serves to prevent optical guiding means 116 from being inserted beyond the preset distance by contacting clamping means 111 from one end.

Clamping means 111 then clamps optical guiding means 116 in place in first introducer means 110, according to the first embodiment. Clamping means 111 serves to ensure that optical guiding means 116 moves with first introducer means 110 as first introducer means 110 is manipulated during the diskectomy procedure for example.

As illustrated in Figure 14A, the first embodiment of a means for clamping, clamping means 111, has a clamping end 111a, midsection 111b and an introducer end 111c. Midsection 111b and introducer end 111c comprise clamp housing 111g. Clamping means 111 can be made of a metal, for example stainless steel, but is preferably made of molded plastic, preferably polycarbonate plastic.

Clamping end 111a comprises a clamp 111a-3 having a clamping head 111a-2, two integrally connected compression legs 111a-3, and side members 111a-7, as illustrated in Figures 14A-14C. Side members 111a-7 are wider than compression legs 111a-3. Clamping head 111a-2, side members 111a-7, and compression legs 111a-3 are preferably molded as one piece. Legs 111a-3 and side members 111a-7 are integrally connected to head 111a-2 at one end while the opposite ends are free.

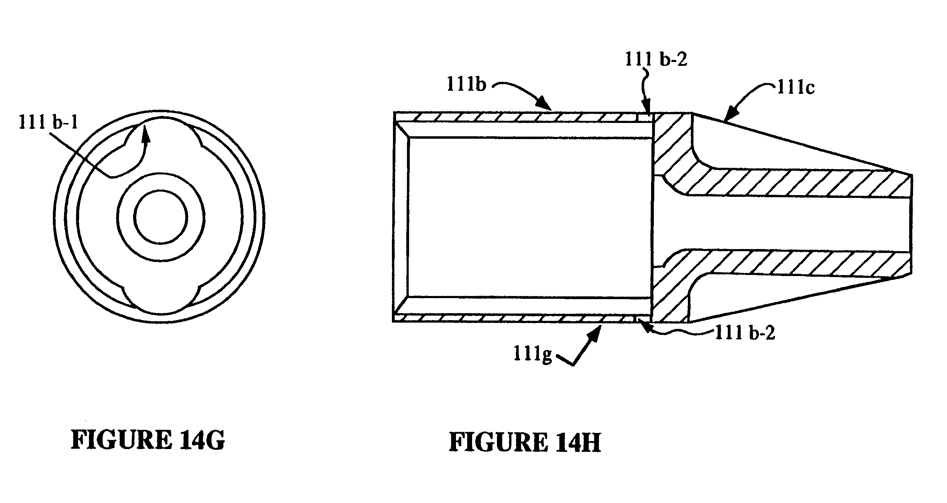

Compression legs 111a-3 and side members 111a-7 of clamp 111a-1 fit within midsection 111b of housing 111g, and each compression leg 111a-3 has a cylindrical boss 111a-4 which projects laterally therefrom, as illustrated in Figures 14A-14F. Bosses 111a-4 fit within internal curved recesses 111b-1 of midsection 111b, as illustrated in Figure 14G, when clamp 111a-1 is fully inserted into housing 111g. Curved recesses 111b-1 serve as cam surfaces while the cylindrical bosses 111a-4 serve as cam followers. Compression legs 111a-3 also each have an engagement ear 111a-5 at the free ends thereof. Engagement ears 111a-5 project laterally out and fit within retention slots 111b-2 in outer housing 111g, as illustrated in Figures 14A-14F and 14H. Retention slots 111b-2 are located in midsection 111b near where midsection 111b and introducer end 111c meet. As clamp 111a-1 is inserted into housing 111g, bosses 111a-4 slide along recesses 111b-1 until engagement ears 111a-5 snap into retention slots 111b-2, thereby fixing the assembly together, as illustrated in Figure 14A. Then housing 111g is rotated while clamping end 111a is held stationary, causing midsection 111b to press in on compression legs 111a-3 with cam and follower action. Engagement ears 111a-5 also move out of retention slots 111b-2 and within midsection 111b as midsection 111b is rotated.

An elastomer 111d is retained by the inner radius of compression legs 111a-3 and side members 111a-7 and is preferably tubular in shape, extending from head 111a-2 to engagement ears 111a-5, as illustrated in Figure 14A. Elastomer 111d is a resilient material, preferably silicone rubber. Elastomer 111d grips or clamps to optical guiding means 116 when housing 111g is rotated. Optical guiding means 116 is inserted into first introducer means 110 through bore 110d to a position indicated by its position indicator means 111h, as illustrated in Figures 18A-18C. Optical guiding means 116 is intended to extend out of end 110a for a distance which is determined by the surgeon to be within the nucleus 18d of the afflicted disc 18b. Compression legs 111a-3 compress elastomer 111d against optical guiding means 116 in the fully rotated, clamped position. The side members 111a-7 prevent radial expansion of the elastomer 111d during the compression. Elastomer 111d grips and prevents axial slippage of optical guiding means 116. The compressed elastomer 111d distributes the clamping force on the optical guiding means in such a manner that the optical transmission characteristics of optical guiding means 116 are not degraded. In addition, elastomer 111d exhibits a large coefficient of friction against optical guiding means 116. This large coefficient of friction minimizes the clamping force required to sustain a given degree of restraining force. Because of the characteristics of elastomer 111d, clamping means 111 is also removable by rotating housing 111g in the opposite direction to release the compression forces without degrading the optical guiding means 116 optical characteristics.

Both clamping means 111 and position indicator means 111h of the first embodiment clamp and grip onto optical guiding means 116 in the same way and clamping means 111 also clamps to stylet 112 in the same fashion. The shapes of housing 111g and position indicator means (111h) housing 111h-1 differ although they comprise similar components. The differences in housing 111g and the housing 111h-1 of position indicator means 111h relate to introducer end 111c. Introducer end 111c is shaped to fit and grip end 110b of first introducer means 110. End 110b is flared as illustrated in Figure 14A and flare grip 111c-1 holds end 110b in place. In the first embodiment of the means for clamping, flared end 110b is bonded into introducer end 111c using an organic adhesive, for example fast bonding adhesives which are compatible with both plastics and metal, like cyanoacrylate adhesives. Clamping means 111 is permanently attached to first introducer means 110 with the adhesive. On the other hand, position indicator means 111h is shaped to facilitate the insertion of the optical guiding means 116, which does not have flared ends, as illustrated in Figures 18A-18C, and is not permanently attached to other instruments.

Clamping means 111 is assembled as follows : First, end 110a of first introducer means 110 is inserted into housing 111g from midsection 111b end until flared end 110b contacts with flared grip 111c-1. End 110b of first introducer means 110 is held in place until bonded with a pre-applied adhesive. Second, elastomer 111d is then inserted within the inner radius of compression legs 111a-3. Third, clamp 111a-1 is inserted into midsection 111b until engagement ears 111a-5 engage with retention slots 111b-2. Housing 111g is not rotated into the clamping position until optical guiding means 116 or stylet 112 is inserted and clamping is necessary.

A second embodiment of the clamping means, instrumentation clamp 700, according to the invention is illustrated in Figures 21A-21F, 22A-22G, 23A, 23B, 24A and 24B. Figure 23B illustrates the assembled instrumentation clamp 700, while Figure 23A is a cross-sectional view of the assembled instrumentation clamp 700 illustrating the internal components of the clamp assembly.

Figure 21A is a cross-sectional view of clamping portion 500. Clamping portion 500 comprises compression legs 503 attached to clamp head 502. Compression legs 503 are preferably integrally connected to clamp head 502 at one end and clamping portion 500 is typically molded as one part. Compression legs 501 each have a projecting ear 503 and a cylindrical-shaped raised portion 504 formed near the free end thereof. The raised portion 504 is located adjacent to projecting ear 503. Clamping portion 500 further comprises extension 505 which projects from the interior of clamp head 502. Extension 505 has a tapered bore which has a larger diameter $d_1$ at the end 505a which protrudes from clamp head 502 than diameter $d_2$ at end 505b where compression legs 501 emanate. The tapered bore facilitates threading a first instrument 800, such as an optical fiber, through bore 505b diameter $d_2$ from end 505a of extension 505.

According the second embodiment, clamp head 502 provides a means for coupling 508 a second instrument 801 to instrumentation clamp 700. Means for coupling 508 can utilize any locking means including a threaded screw lock, a snap lock, bayonet-type lock, or a friction fitted lock, which are known in the art, or preferably a luer lock. Second instrument 801 can have the complementary fitting to clamp 700. In the preferred embodiment, the means for coupling 508 incorporates thread receiving grooves 508-1 on the interior surface of clamp head 502. The thread receiving grooves 508-1 receive the threads 802-1 of a female luer fitting 802 of a second instrument 801, as illustrated in Figure 21A. Instrumentation clamp 700 can be made of engineering plastic, such as ABS or polycarbonate plastic. For the preferred embodiment, instrumentation clamp 700 is made of a polycarbonate plastic. For the invention, the means for coupling 508 can be either the male lock fitting or the female lock fitting, while the male lock fitting is preferred for instrumentation clamp 700.

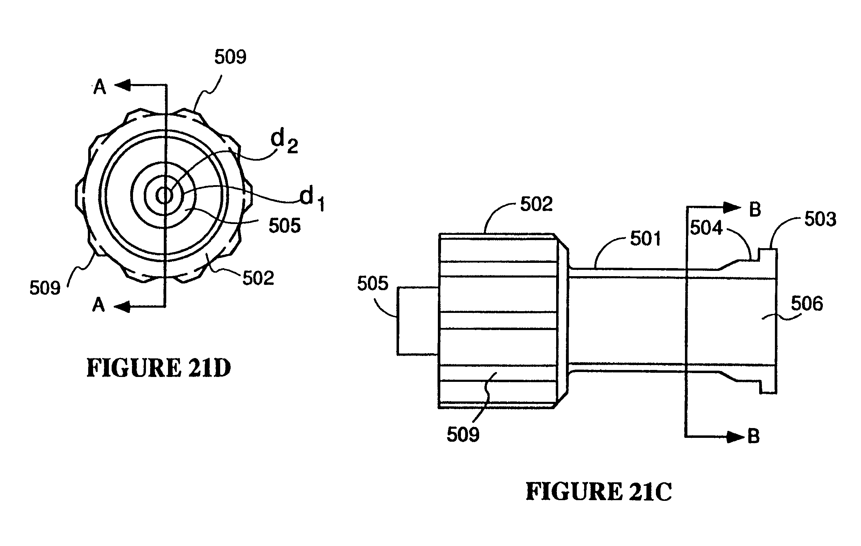

Figure 21C and 21E are side views of clamping portion 500 of the second embodiment. Figure 21C illustrates a side view of compression legs 501. In Figure 21E, a front view of one compression leg 501 is illustrated along with a side view of compression extensions 506. Compression extensions 506 facilitate holding resilient tube 507 along with compression legs 501. Compression legs 501 and compression extensions 506 have internal and external curvature, as illustrated in Figure 21B. Although Figure 21B illustrates only two compression legs 501, in other embodiments, there can be more than two. As the number of compression legs 501 increases, the size of the compression extensions 506 decreases. Compression legs 501 are designed to flex inwardly, as shown by arrows in Figure 21A, toward a resilient tube 507 (not shown). Resilient tube 507 is illustrated in cross section in Figure 23A and is similar to elastomer 111d of the first embodiment of the means for clamping. Resilient tube 507 has a central bore 507a which is aligned with bore end 505b having diameter $d_2$ in clamp head 502.

Figure 21D illustrates an end view of clamp head 502 wherein the tapered bore having diameters $d_1$ and $d_2$ through projection 505 are shown in relation to clamp head 502. Also shown are a plurality of ribs 509 on the surface of clamp head 502. Ribs 509 serve as gripping and handling members of clamp 700.

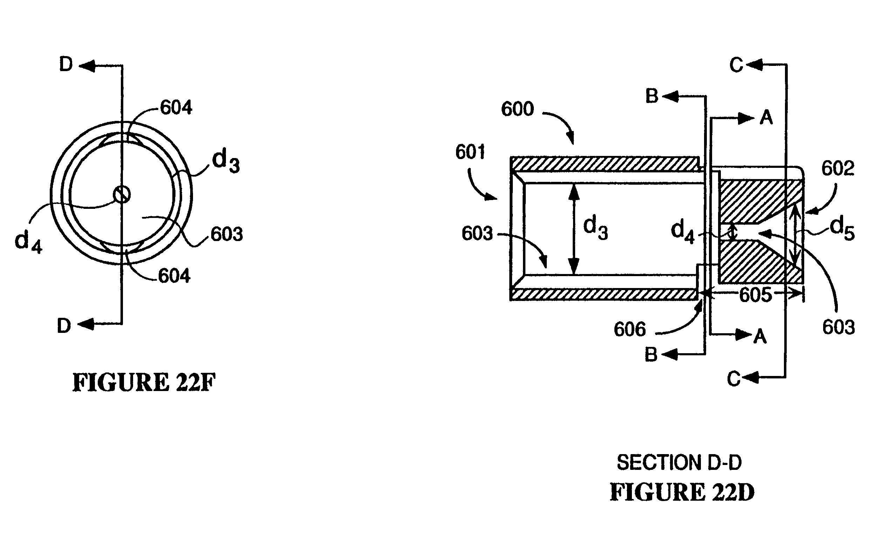

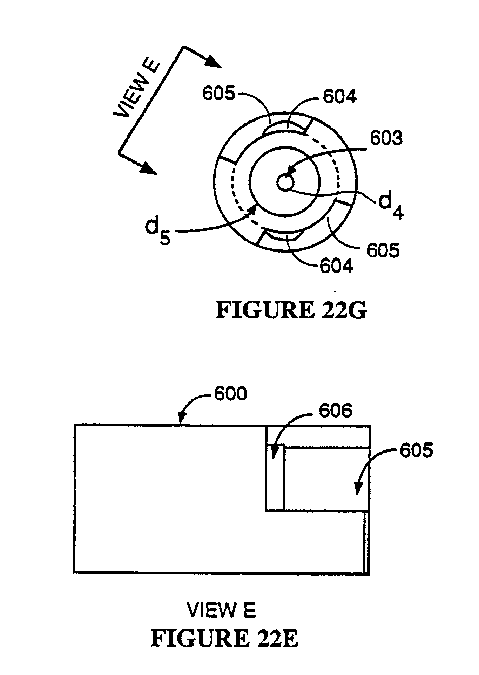

Figure 22D illustrates a cross sectional view of clamp housing 600 of the second embodiment. Clamp housing 600 is essentially a cylindrical tube having a bore 603 and comprises a clamp receiving end 601 and a clamp securing end 602. Clamp receiving end is illustrated in Figure 22F. Bore 603 having diameter $d_3$ extends along the length of clamp receiving end 601 and narrows to diameter $d_4$ in clamp securing end 602. Bore 603 flares out to diameter $d_5$ at the other end of clamp securing end 602 (see Figures 22D, 22F and 22G). The tapering of bore 603 facilitates threading a first instrument into clamp 700 from clamp securing end 602. The end view of clamping end 601, as illustrated in Figure 22F, also illustrates recesses 604 for receiving raised portions 504 and projecting ears 503 on compression legs 501. Recesses 604 extend along the length of the interior surface of clamp housing 600 and have a radius of curvature designed to match the radius of curvature of raised portions 504.

Figure 22G illustrates an end view of clamp securing end 602. Clamp securing end 602 has cutout portions 605 which extend approximately from an edge of each recess 604 for a distance around the perimeter of end 602 and along the length of clamp securing end 602. Recesses 604 are generally aligned with one edge of cutouts 605 and each respective recess 604 and cutout 605 are directly opposite the other in the preferred embodiment. Through each cutout 605 is a projecting ear receiving slot 606 which extends from edge to edge of cutout 605 near where clamp securing end 602 ends and clamp receiving end 601 begins. Receiving slots 606 are illustrated in Figures 22D and 22E.

Figure 22B is a cross sectional view of clamp housing 600 cut along line B-B indicated Figure 22D. Cutouts 605 are not cross-hatched and are shown in relation to recesses 604 in Figure 22B.

Figure 22A is a cross sectional view of clamp housing 600 cut along line A-A in Figure 22D. Figure 22A illustrates the cross section of clamp housing 600 directly opposite to that of the cross section of clamp housing 600 in Figure 22B where recesses 604 end in clamp receiving end 601 and cutouts 605 begin in clamp securing end 602.

Figure 22C is a cross-sectional view of clamp securing end 602 cut along line C-C of Figure 22D. Figure 22C illustrates the tapering of bore 603 from diameter $d_5$ to diameter $d_4$ in clamp securing end 602. Also shown are cutouts 605 and recesses 604.

To assemble clamp 700, resilient tube 507, made from a resilient elastomer, such as silicone rubber, for example, is placed within the interior curvature of compression legs 501 and compression extensions 506 so that bore 507a in resilient tube 507 is aligned with bore 505b of clamping portion 500. Clamp housing 600 is snap coupled to clamping portion 500, after resilient tube 507 is installed. First, projecting ears 503 of compression legs 501 are aligned with recesses 604 of clamp housing 600. Compression legs 501 are compressed inwardly until projecting ears 503 fit into clamp housing 600 within recesses 604. Second, while holding clamping portion 500 by clamp head 502 and holding clamp housing 600 by clamp securing end 602, clamp housing 600 is slid over compression legs 501 until projecting ears 503 are received by receiving slots 606. Projecting ears 503 emanate from receiving slots 606 and curved portions 504 are received by recesses 604 when instrumentation clamp 700 is in an open position.

To clamp to a first instrument, preferably an optical fiber 800, one end of optical fiber 800 is inserted into bore 603 from clamp securing end 602. Optical fiber 800 is threaded through bore 603 diameter $d_5$ and through end 505b of clamping portion 500 to emanate out end 505a of extension 505 in clamping portion 500. When the optical fiber 800 is inserted to the desired distance through clamp 700, optical fiber 800 is clamped in place by rotating clamp housing 600 while clamping portion 500 is held stationary until projecting ears 503 slide from one end of receiving slots 606 to the other end of receiving slots 606. When clamp housing 600 is rotated, raised portions 504 exit recesses 604, thereby causing compression legs 501 to compress against resilient tube 507 by cam and follower action. Instrumentation clamp 700 is now in a closed position. The compressive force generated when clamp housing 600 is rotated closed is sufficient to hold optical fiber 800 securely with minimal slippage and minimal deformation of the optical fiber's transmission characteristics.

To temporarily couple a second instrument 801 to clamp 700, second instrument 801, having a central bore 803, preferably has a threaded luer female fitting 802 at one end with bore 803a therethrough to receive extension 505 from clamping portion 500 as threads 802-1 are received by thread receiving grooves 508-1. Moreover, second instrument 801 preferably has bore 803b which is narrower than bore 803a and is generally aligned therewith. A first instrument 800, such as an optical fiber, can be threaded through clamp 700 and through bore 803 of second instrument 801 for simultaneous manipulation.

Second instrument 801 can be coupled to clamp 700 using means for coupling 508 prior to the insertion and clamping of first instrument 800. Once second instrument 801 is coupled, optical fiber 800 is threaded through bore 603 at end 602 until it emanates from bore 803b of second instrument 801 to a desired distance. Once threaded, clamp housing 600 is rotated relative to clamping portion 500 and second instrument 801 to a closed position, as described above, wherein projecting ears 503 slide to opposite ends of receiving slots 606 and raised portions 504 exit recesses 604 within clamp housing 600. At this point, optical fiber 800 is securely clamped with minimal slippage and deformation within second instrument 801 for simultaneous manipulation. Figure 24A illustrates the simultaneous clamping of optical fiber 800 and coupling of second instrument 801 to clamp 700 according to the preferred embodiment. Figure 24B illustrates the clamping of the preferred embodiment in cross section. Gripping handle 804 is attached to the periphery of second instrument 801 and facilitates manipulation of second instrument 801.

According to a preferred implementation of instrumentation clamp 700, a position indicator means is no longer necessary. Once the desired length of optical guiding means 116 is determined, clamp 700 clamps optical guiding means 116 at a predetermined position in the same way that position indicator means 111h was used. The clamped optical guiding means 116 is threaded through an introducer means to extend a predetermined distance out of the introducer means. In this embodiment, clamp 700 is not permanently attached to the introducer means and is a self-contained means for clamping according to the preferred embodiment. The introducer means is adapted to couple to clamp 700 by coupling means 508, as described above, using the preferred luer lock coupling mechanism 508, as illustrated in Figures 23A, 24A and 24B. The instrumentation clamp 700 provides a temporary and secure coupling to the introducer means and clamp 700 can be readily disconnected and reused. The introducer means of the invention can be any fiber introducing instrument and the instrument and clamp are not limited to use in percutaneous diskectomy. For example, the introducer means and means for clamping of the first and second embodiments can be used in orthopedic surgery.

Fourth, using laser energy from the first laser means through first optical guiding means 116, some of the nucleus pulposus within nucleus 18d is vaporized to create a first vaporized area in the nucleus 18d of the herniated disc 18b. The first vaporized area provides a space or cavity in the nucleus pulposus into which nucleus pulposus from the herniated area 18c can fill and thereby contract away from nerve root 18e. First optical guiding means 116 along with first introducer means 110 are removed from cannula 104 when the vaporization step is complete.

According to the invention, a second vaporization step is included. According to the preferred embodiment, a second introducer means 130 is inserted into cannula 104 to contact the first vaporized area.

Second introducer means 130 is preferably 14 gauge along its length and has a 17 gauge tip 130a. Second introducer means 130 is metal, preferably type 304 stainless steel, No. 3 temper (full hard). Moreover, the opening in tip 130a of second introducer means 130 is formed differently from first introducer means 110, as illustrated in Figure 15 and in an enlarged view illustrated in Figure 16. Rather than opening 130a-1being perpendicular to the longitudinal axis of the tubular member as is shown for first introducer means, opening 130a-1 at end 130a is curved relative to the longitudinal axis. Curved end 130a is not flared out nor wider than the 14 gauge portion of the tubular member. As a result, curved end 130a of second introducer means 130 need not be wider in diameter than first introducer means 110. In the preferred embodiment, second introducer means 130 has the same inner and outer diameter as first introducer means 110 and has a curvature at end 130a within that outer diameter. Therefore, second introducer means 130 fits within cannula 104 in the same way first introducer means 110 fits within cannula 104. Cannula 104 remains in the patient's body to receive second introducer means 130 for the second vaporization step according to the preferred embodiment, as described below.

Fifth, the curved end 130a of second introducer means 130 enters the nucleus 18d and contacts the first vaporized area when second introducer means 130 is inserted into cannula 104. One end of a second optical guiding means 132 is inserted through a central bore 130d, of second introducer means 130 to emanate from opening 130a-1 into the first vaporized area at the formed end 130a of second introducer means 130, as illustrated in the enlarged view in Figure 16. Second optical guiding means 132 has a position indicator means which is the same as position indicator means 111h on first optical guiding means 116 according to the first embodiment. The position indicator means on second optical guiding means 132 contacts with clamping means 131 in the same way as described above for first introducer means 110 and position indicator means 111h. Clamping means 131 and 111 are essentially the same and clamping means 131 is illustrated in Figure 15. Alternatively, instrumentation clamp 700 of the second embodiment can be used for clamping the optical guiding means 116, 132 for both vaporization steps. Clamp 700 eliminates the need for two clamping means 111 and 131, permanently associated with first and second introducer means 110 and 130, and two position indicator means. The first and second introducer tubes only need to be adapted to accommodate the means for coupling 508 of the second embodiment instrumentation clamp 700.

When end 132a of second optical guiding means 132 emanates from opening 130a-1 of curved end 130a on second introducer means 130, end 132a of second optical guiding means 132 is deflected off the longitudinal axis of the second optical guiding means 132. The amount which second optical guiding means 132 is deflected is dependent upon the radius of curvature of end 130a of second introducer means 130.

The considerations made when determining what radius of curvature to use at least depended on several factors, according to the invention. First, the minimum radius of curvature should be so formed at the tip of an introducer means so that the curved introducer means still fits within cannula 104. Second, optical guiding means 116 or 132, for example an optical fiber, should deflect with uniform curvature to achieve a minimal loss of laser light guiding efficiency. Third, the radius of curvature of the introducer means allowable and the diameter of the optical guiding means allowable are mutually dependent. According to the preferred embodiment, the radius of curvature is 0.45 which deflects second optical guiding means 132 about 17° from the longitudinal axis when second optical guiding means 132 is a 400$\mu$m optical fiber. Second optical guiding means 132 can be deflected between the range of 1° to 30° by curved end 130a of second introducer means 130, for the preferred embodiment.

Once second optical guiding means 132 is in place and positioned so that it extends out of curved end 130a of second introducer means 130 for a distance, as predetermined by the surgeon, optical guiding means 132 is clamped in place by clamping means 131 in much the same way as described previously for clamping means 111 of the first embodiment, or alternatively, can be clamped in place as described for instrumentation clamp 700 of the second embodiment. Therefore, clamping of second optical guiding means 132 to second introducer means 130 allows second optical guiding means to be manipulated as second introducer means 130 is manipulated. An end of second optical guiding means 132 opposite to the deflected end is attached to a second laser means. Light energy from the second laser means is guided by second optical guiding means 132 into the nucleus 18d to vaporize nucleus pulposus and create a second vaporized area within nucleus 18d. The second vaporized area of the preferred embodiment is larger than the first vaporized area and the larger area is created by the deflected beam emanating from deflected end 132a of second optical guiding means 132 during this vaporization step. Manipulation of second introducer means 130 with second optical guiding means 132 clamped thereto will cause manipulation of the deflected beam as well.

According to the invention, when the laser beam is applied generally along a line 30-1 to a herniated disc area, the line or path that the laser beam takes is illustrated by example in Figure 19A. Line 30-1 is obtained by moving first introducer means 110 having first optical guiding means 116 disposed therethrough axially within cannula 104. Since laser beams according to the invention are divergent and emanate in a 15° cone from the guiding means, the line or path defined by the divergent beam is described as a single overall direction the laser beam travels, as illustrated by arrow A in Figure 19A. Line 30-2 to a herniated disc area can also be the path of the laser beam, as illustrated in Figure 19B. Line 30-2 to a herniated disc area is obtained with second introducer means 130 having second optical guiding means 132 disposed therethrough, being deflected off the longitudinal axis by curved end 130a. The laser beam guided through deflected second optical guiding means 132 is applied along line 30-2.

The laser beam can be applied along a line 31-1, as illustrated in Figure 20A. Line 31-1 is different from line 30-1, as illustrated in Figures 19A and 20A, and the difference is at least due to shape of straight first introducer means 110 relative to the shape of curved second introducer means 130. Line 31-1 is obtained by guiding a laser beam along second optical guiding means 132 while second optical guiding means 132 is disposed in curved second introducer means 130.

When the laser beam is applied along line 31-2, line 31-2 is different from line 30-1 and line 30-2, as illustrated in Figures 20A and 20B. Line 31-2 is obtained by guiding a laser beam along second optical guiding means 132 while second optical guiding means is disposed in curved second introducer means 130. Moreover, curved end 130a of second introducer means 130 is inserted into cannula 104 at a position rotated a distance from line 30-2. Line 31-2 is at an angle to both line 30-1 and line 30-2.

When the laser beam is applied along a line 31-3, line 31-3 is different from line 30-1 and line 30-2, as illustrated in Figure 20C. Line 31-3 is at an angle to line 30-1 and parallel to line 30-2. Line 31-3 is obtained by guiding a laser beam along second optical guiding means 132 while second optical guiding means 132 is disposed in curved second introducer means 130 and second introducer means 130 is moved axially a distance within cannula 104 along the path followed by second introducer means 130 for line 30-2.

According to the preferred embodiment, second introducer means 130 having curved tip 130a can be moved axially within cannula 104 while the laser beam applied to the nucleus from deflected end 132a of second optical guiding means is at an angle to the direction of movement. Moreover, second introducer means 130 having second optical guiding means 132 disposed therethrough can be rotated to any distance through 360 degrees to apply the laser beam in an arc up to 360 degrees. The laser beam from second introducer means 130 can be applied along a plurality of lines through 360 degrees or less and each line would be at an angle to the previous line. Second introducer means 130 can be moved axially within cannula 104 while being rotated through 360 degrees at least one time and preferably several times during the second vaporization step. The deflected beam from second optical guiding means 132 and the movement increase the amount of nucleus pulposus vaporized in the second vaporized area. Second introducer means 130 having curved end 130a articulates second optical guiding means 132 to increase the amount of nucleus pulposus which can be vaporized. Second introducer means 130 articulates the second optical guiding means 132 in a static way because second introducer means 130 has one predetermined curved end 130a which will deflect second optical guiding means 132 in one way and to a fixed degree. Different introducer means having different radii of curvature can be used in addition to second introducer means 130 and still be within the scope of the invention.

On the other hand, variable articulators are known in the art which articulate optical fibers in numerous ways and to different degrees in endoscopic procedures. Variable or dynamic articulators of the relevant art are much larger in diameter and require much larger paths along which they are manipulated. As a result, variable articulators are used in endoscopic surgery through preexisting body cavities. Second introducer means 130 is a static articulator which can be manipulated within much smaller paths than the variable articulators because of second introducer means 130 design and construction. Therefore, static articulator or second introducer means 130 of the present invention works well in percutaneous procedures while variable articulators do not. Also, second introducer means 130 can vaporize a larger given area than straight first introducer means 110 when each is manipulated along the same small path or cannula 104, as described above.

Second optical guiding means 132 may be of the same construction as first optical guiding means 116 or may be different. In the preferred embodiment, second optical guiding means 132 has the same construction as first optical guiding means 116, and first optical guiding means 116 preferably is reused as second optical guiding means 132 during the second vaporization step in a single diskectomy procedure. Reuse of an optical fiber in multiple diskectomy procedures is not recommended. In one embodiment, an optical fiber is used as first optical guiding means 116. The optical fiber is preferably used as second optical guiding means 132, or another optical fiber may be used. The optical fibers preferably have the same construction but they can have different constructions, depending upon the application. For the invention, the optical fibers are for single use and disposable. In another embodiment, a hollow optical waveguide is used as first optical guiding means 116. A hollow optical waveguide can be used as second optical guiding means 132, as well, with slight modification to one end of the optical waveguide to adapt it to formed end 130a of second introducer means 130. The optical fibers are preferred over the hollow optical waveguides for the present invention. Alternatively, one optical guiding means can be an optical fiber, while the other optical guiding means can be an optical wave guide in still another embodiment. The particular optical guiding means used for the different embodiments will depend on the application and the laser means which is also used.

The first and second laser means are typically the same laser which emits one wavelength of light. Alternatively, two wavelengths of laser light or two different lasers may be used to produce a laser beam for vaporizing nucleus pulposus. According to the present invention, only one laser is necessary. The laser system used for percutaneous diskectomy according to the present invention can emit energy in the temporal continuous mode or pulse mode in the ultraviolet, visible and infrared ranges of the electromagnetic spectrum. Table I lists the lasers and the associated wavelengths for use in percutaneous diskectomies according to the invention. For example, a Nd : YAG laser which emits energy at 1064 nm can be modified by second harmonic generation to create a laser beam at another wavelength. In the preferred embodiment, a Nd : YAG laser which emits light at 1064 nm is coupled with a frequency doubler to generate a laser beam at 532 nm. For the preferred embodiment, a solid state media is used as a frequency doubler, in particular a potassium, titanyl phosphate crystal (KTP), to create a laser system according to the present invention which is usable with either the first or second laser means, or both. The laser system of the preferred embodiment, has been used for other percutaneous surgical procedures in the areas of gynecology, urology, dermatology, gastroenterology, otorhinolaryngology, and other neurosurgeries, but has not been used for applying a laser beam in percutaneous diskectomies, according to the present invention. The laser system of the preferred embodiment is known in the art as KTP/532™ Surgical Laser System.

## TABLE I

### LIST OF LASERS FOR USE IN PERCUTANEOUS DISKECTOMY

| Laser Type | Wavelength |
|---|---|
| | (Nanometers or Micrometers) |
| $CO_2$ | 10.6 $\mu$m |
| CO | 5, 7 $\mu$m |
| Erbium:YAG | 2.94 $\mu$m |
| Holmium:YAG | 1950 nm, 2150 nm |
| Krypton | 647 nm |
| Argon | 488 nm, 514.5 nm |
| Dye Lasers | 350 nm, 1000 nm |
| Nd:YAG | 1320 nm |
| Nd:YAG (frequency doubled) | 532 nm, 660 nm |
| Nd:YAG (frequency tripled) | 354.7 nm, 440 nm |
| Nd:YAG (frequency quadrupled) | 266 nm, 330 nm |
| **Tunable Lasers:** | |
| $Co:MgF_2$ | 1.75 $\mu$m, 2.5 $\mu$m |
| Ti:Sapphire | 660 nm, 990 nm |
| Ti:Sapphire (frequency doubled) | 330 nm, 495 nm |
| Alexandrite | 730 nm, 780 nm |
| Alexandrite (frequency doubled) | 365 nm, 390 nm |
| **Excimer Lasers:** | |
| Xenon Chloride | 308 nm |
| Xenon Fluoride | 248 nm |
| Argon Fluoride | 193 nm |
| Krypton Fluoride | 248 nm |

The laser system according to the present invention should be any laser which emits laser energy that is absorbed by body tissue. The first and second laser means are preferably one laser system which is used in both the first and second vaporization steps.

Any laser system used in accordance with the present invention that emits a laser beam in the ultraviolet or visible range of the electromagnetic spectrum can be used in conjunction with optical guiding means 116 and 132 of the preferred embodiment, namely an optical fiber. Any laser system that emits a laser beam in the infrared range of the electromagnetic spectrum can be used in conjunction with a hollow optical waveguide. Therefore, the Argon laser for example, or preferably Nd : YAG laser modified by second harmonic generation will emit a laser beam that is conducted by an optical fiber, according to the present invention. The $CO_2$ laser will emit a laser beam that is conducted by a hollow optical waveguide, according to the present invention. In another embodiment, two different wavelengths of laser light or two different lasers are used, one laser which typically uses an optical fiber to conduct its laser beam and one laser which typically uses a hollow optical waveguide to conduct its laser beam, as described above.

After the second vaporization step according to the preferred embodiment, second optical guiding means 132 and second introducer means 130 are removed from cannula 104. In the preferred embodiment, cannula 104 is also removed and the entry point through the skin is covered with a sterile bandage. The patient is then allowed to leave the hospital and recuperate at home under minimal restrictions or requirements.

Alternatively, in still another embodiment an irrigation/aspiration cannula 150, is inserted into cannula 104 after second introducer means 130 and second optical guiding means 132 are removed. Irrigation/ aspiration cannula 150 is preferably 15 gauge along its length and has a 17 gauge tip 150a, as illustrated in Figure 17. Cannula 150 is used to evacuate the second vaporization area so that the second vaporization area can be further cleansed in the unlikely event that loose fragments or debris might be present. A vacuum suction device is attached to end 150a of cannula 150 and the area is aspirated, before cannula 104 is removed.

The means for inserting instrumentation necessary for percutaneous diskectomy using a laser can be packaged in a kit and sold, for example, for single use or multiple use. The kit may contain probe 100, straight cannula 104, curved cannula 106, first introducer means 110, second introducer means 130, stylet 112, cannula 150 and tools such as a marking pen, scalpel with blade, measuring scale and a locking stabilizer 105. The kit may contain all these items or some of them. For example, the kit may contain instrumentation clamp 700, according to the second embodiment. Furthermore, optical guiding means 116 and 132 may be included. The laser system according to the preferred and exemplary embodiments may be supplied separately also.

While the invention has been described in connection with several exemplary embodiments, it will be understood that many modifications will be apparent to those of ordinary skill in the art, while still being within the intended scope of the present invention.

## Claims

1. A clamping device for clamping an instrument comprising :
   a resilient tube for grasping such an instrument ; and
   means operatively associated with the resilient tube for providing that upon force being applied to the resilient tube, such an instrument is substantially secured without substantial deformation of such an instrument.

2. A device as claimed in claim 1 wherein the means operatively associated with the resilient tube comprises a clamp disposed around the resilient tube, the clamp being actuatable to selectively apply force to the resilient tube.

3. A device as claimed in claim 2 wherein the means operatively associated with the resilient tube comprise a housing which encloses the resilient tube.

4. A device as claimed in claim 3 wherein the clamp has a plurality of compression legs integrally connected to a clamp head at one end and being movable at an opposite end, wherein each leg has protrusion means for retaining the clamp in the housing, and wherein the plurality of compression legs surrounds the resilient tube.

5. A device as claimed in claim 4 wherein the housing has receiving means for receiving the protrusion means when the clamp is inserted, the receiving means and the protrusion means cooperating to apply force to the resilient tube.

6. A device as claimed in claim 5 wherein rotation of the housing with respect to the clamp in a first direction causes the cooperation between the receiving means and the protrusion means to secure such an instrument.

7. A device as claimed in claim 5 wherein the rotation of the housing in a first direction causes the plurality of compression legs to move toward the resilient tube to compress the resilient tube to secure such an instrument.

8. A device as claimed in claim 8 wherein receiving means comprises a retention slot and a curved recess on an inside radius of the housing for each of the compression legs.

9. A device as claimed in claim 8 wherein the protrusion means comprises a substantially cylindrical boss and an engagement projection near the movable end of each of the compression legs, wherein the engagement projection and the cylindrical boss extend radially toward the housing, and wherein the engagement projection extends further than the cylindrical boss extends.

10. A device as claimed in claim 9 wherein the curved recess receives both the cylindrical boss and the engagement projection while the clamp is being inserted into the housing, and the retention slot receives the engagement projection to retain the clamp in the housing.

11. A device as claimed in any one of claims 6-10 wherein rotation of the housing with respect to the clamp in a second direction causes the cooperation between the receiving means and the protrusion means to release such an instrument.

12. A clamping device for clamping an instrument comprising a first part permanently associated with an elongate tube for inserting such an instrument percutaneously, the first part having a first bore therethrough, and a second part for receiving such an instrument, the second part having a second bore therethrough, the second part being permanently affixed and adjacent to the first part, so that the first bore is generally aligned with the second bore.

13. A device as claimed in claim 12 wherein the first part is made from a material which can be readily sterilized with minimal distortion.

14. A clamping device as claimed in claim 12 wherein the second part comprises :
    a resilient tube for grasping such an instrument ;
    a clamp surrounding the resilient tube for compressing the resilient tube ; and
    a housing operatively associated with the clamp, the housing being rotatable with respect to the clamp to force the clamp to compress the resilient tube.

15. A device as claimed in any one of claims 3-11 and 14 wherein the clamp and the housing are made from a material which can be readily sterilized with minimal distortion.

16. A device as claimed in any preceding claim comprising an instrument, the instrument being an optical fiber or a stylet.

17. An instrumentation clamping device comprising :
    a resilient tube having a central bore for holding a first instrument ;
    a clamping portion having holding means for holding the resilient tube and having means for coupling to a second instrument ; and
    a clamp housing operatively secured to the means for holding the resilient tube, the clamp housing selectively compressing the means for holding against the resilient tube.

18. A device as claimed in claim 17 wherein the clamp housing is rotatable with respect to the clamping portion and rotation of the clamp housing in one direction selectively compresses the holding means.

19. A device as claimed in claim 17 wherein the holding means is a plurality of compression legs having an inside radius for receiving a curvature of said resilient tube.

20. A device as claimed in claim 19 wherein at least two of the compression legs have means for engaging the clamping portion with the clamp housing.

21. A device as claimed in claim 20, wherein the clamp housing has a plurality of recesses in an interior surface thereof and a plurality of retention slots through clamp housing near a first end thereof, the recesses extending from a second end of the clamp housing and terminating adjacent a respective retention slot.

22. A device as claimed in claim 21 wherein the means for engaging are received in the recesses and the retention slots.

23. A device as claimed in claim 22 wherein the means for engaging comprises a first plurality of projections located on an end of the at least two compression legs, and a second plurality of projections located adjacent to the first plurality of projections, the first plurality of projections projecting further out from the compression legs away from the resilient tube than the second plurality of projections.

24. A device as claimed in claim 23 wherein the projections of the first plurality of projections are received through the slots of the plurality of retention slots.

25. A device as claimed in any one of claims 17-24 wherein the means for coupling is a threaded locking mechanism.

26. A device as claimed in claim 25 wherein the threaded locking mechanism is a luer lock, and such a second instrument has one luer fitting and the instrumentation clamp device has the complementary luer fitting.

27. A device as claimed in any one of claims 17-24 wherein the means for coupling provides a temporary connection between the instrumentation clamp device and such a second instrument.

28. A device as claimed in any one of claims 17-27 comprising a first instrument, the first instrument being an optical fiber.

29. A device as claimed in any one of claims 1-11 & 14-28 wherein the resilient tube is of silicone rubber or other elastomer.

FIGURE 2

FIGURE 1

**FIGURE 3A**

**FIGURE 3B**

EP 0 439 263 A1

EP 0 439 263 A1

FIGURE 4
PRIOR ART

FIGURE 5

18

100c

100

100a

100b

**FIGURE 6**

EP 0 439 263 A1

104b

102b

104

102a

104a

105

**FIGURE 9**

100

18a

18b

**FIGURE 7**

18d

18b

18a

100

18e

18c

**FIGURE 8**

## FIGURE 10 A

103b

103 b-2

103 b-2

103 b-1

104

B

B

## FIGURE 10 C

102

D

D

103 a

## FIGURE 10 B

103 b-2

103 b-1

103 b

103 b-2

B-B

## FIGURE 10 D

103 a-2

103 a-1

103 a

103 a-2

D-D

EP 0 439 263 A1

**FIGURE 10E**

**FIGURE 10F**

F-F

**FIGURE 11**

**FIGURE 17**

EP 0 439 263 A1

FIGURE 12

FIGURE 13

FIGURE 15

**FIGURE 14A**

FIGURE 16

130

130d

130a

132

132a

FIGURE 14B
SECTION B-B

111a-5
111a-3
111a-7
111a-3
111a-5

111a-4

111a-7
111d
111a-4
111a-7

## FIGURE 14C

111 a-7    111 a-4    111 a-5

111 a-3

## FIGURE 14D

111 a-7

111 a-5

111 a-4

111 a-3

111 a-5

111 a-4

## FIGURE 14E

F

111 a-5

111 a-4

F

## FIGURE 14F
### SECTION F-F

111 a-5

111 a-4

EP 0 439 263 A1

111c

111 b-2

111b

111 b-2

111g

**FIGURE 14H**

111 b-1

**FIGURE 14G**

## FIGURE 18A

## FIGURE 18B

111h

END VIEW

## FIGURE 18C

111h

EP 0 439 263 A1

FIGURE 19A

116

15°

30-1

FIGURE 19B

132

30-2

15°

## FIGURE 20A

## FIGURE 20B

## FIGURE 20C

SECTION A-A

**FIGURE 21A**

SECTION B-B

**FIGURE 21B**

EP 0 439 263 A1

EP 0 439 263 A1

**FIGURE 21D**

**FIGURE 21C**

FIGURE 21F

FIGURE 23B

FIGURE 21E

FIGURE 23A

SECTION B-B

**FIGURE 22B**

SECTION A-A

**FIGURE 22A**

SECTION C-C

**FIGURE 22C**

EP 0 439 263 A1

SECTION D-D
**FIGURE 22D**

**FIGURE 22F**

FIGURE 22G

VIEW E

FIGURE 22E

**FIGURE 24A**

EP 0 439 263 A1

**FIGURE 24B**

EP 0 439 263 A1

**European Patent Office**

## EUROPEAN SEARCH REPORT

Application Number

EP 91 30 0218

### DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | US-A-4 787 884 (GOLDBERG)<br>* Column 4, lines 25-49; figure 5 * | 1-3,12-18,25,26 | A 61 B 17/36<br>A 61 B 17/22 |
| X | EP-A-0 069 389 (SUMITOMO)<br>* Page 5, line 20 - page 6, line 19; figure 4 * | 1-3,12-18,27-29 | |
| Y | | 4-7 | |
| Y | DE-A-3 603 345 (OLYMPUS)<br>* Page 7, line 2 - page 8, line 17; figure 3 * | 4-7 | |
|  |  |  | **TECHNICAL FIELDS SEARCHED (Int. Cl.5)**<br><br>A 61 B<br>A 61 M |

The present search report has been drawn up for all claims

| Place of search | Date of completion of the search | Examiner |
|---|---|---|
| THE HAGUE | 12-04-1991 | MOERS R.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)